# NEUE EUROPÄISCHE PATENTSCHRIFT

(11) **EP 1 202 952 B2**
(45) Veröffentlichungstag und Bekanntmachung des Hinweises auf die Entscheidung über den Einspruch: **09.07.2014**
(45) Hinweis auf die Patenterteilung: 18.05.2005
(21) Anmeldenummer: 00943936.5
(22) Anmeldetag: 29.06.2000
(51) Int. Cl.: C07C 209/26, C07C 227/08, C07D 295/02, C07D 295/14

(54) **VERFAHREN ZUR HERSTELLUNG VON AMINEN DURCH HOMOGEN KATALYSIERTE REDUKTIVE AMINIERUNG VON CARBONYLVERBINDUNGEN**
METHOD FOR PRODUCING AMINES BY HOMOGENEOUSLY CATALYZED REDUCTIVE AMINATION OF CARBONYL COMPOUNDS
PROCEDE DE PREPARATION D'AMINES PAR AMINATION PAR REDUCTION A CATALYSE HOMOGENE DE COMPOSES CARBONYLE

(30) Priorität: 17.07.1999 DE 19933611
(43) Veröffentlichungstag der Anmeldung: 08.05.2002
(73) Patentinhaber: Evonik Degussa GmbH, 45128 Essen (DE)
(72) Erfinder: RIERMEIER, Thomas, D-65439 Flörsheim (DE); HAACK, Karl-Josef, D-65527 Niedernhausen (DE); DINGERDISSEN, Uwe, D-64342 Seeheim-Jugenheim (DE); BÖRNER, Armin, D-18055 Rostock (DE); TARAROV, Vitali, Moskau, 113525 (RU); KADYROV, Renat, D-65931 Frankfurt am Main (DE)
(74) Vertreter: Godemeyer Blum Lenze - werkpatent
(86) Internationale Anmeldenummer: PCT/EP2000/006056
(87) Internationale Veröffentlichungsnummer: WO 2001/005741

(56) Entgegenhaltungen:
- EP-A- 0 893 430
- EP-A1- 1 078 915
- WO-A1-95/21151
- US-A- 2 879 293
- L. MARKO ET AL.: "Homogeneous Reductive Amination with Cobalt and Rhodium Carbonyls as Catalysts" JOURNAL OF ORGANOMETALLIC CHEMISTRY., Bd. 81, 1974, Seiten 411-414, XP002149510 ELSEVIER-SEQUOIA S.A. LAUSANNE., CH ISSN: 0022-328X in der Anmeldung erwähnt
- DATABASE WPI Section Ch, Week 200011 Derwent Publications Ltd., London, GB; Class E19, AN 2000-119544 XP002149513 & JP 11 343269 A (KURARAY CO LTD), 14. Dezember 1999 (1999-12-14) in der Anmeldung erwähnt
- H.-U. BLASER ET AL.: 'Iridium Ferrocenyl Diphosphine Catalyzed Enantioselective Reductive Alkylation of a Hindered Aniline' SYNLETT 1999, Seiten 867 - 868
- TARAROV, V.I. ET AL.: 'Phosphines versus phosphinites as ligands in the rhodium catalyzed asymmetric hydrogenation of imines: a systematic study' TETRAHEDRON: ASYMMETRY Nr. 10, 1999, Seiten 4009 - 4015
- J. MARCH: 'Advanced Organic Chemistry', Bd. 4, 1992, JOHN WILEY & SONS, NEW YORK Seiten 898 - 900
- K. PETER ET AL.: 'Organic Chemistry', 1987, W.H. FREEMAN & COMPANY Seiten 961 - 962
- ALDEA, R. ET AL.: 'Ruthenium clay catalyzed reduction of x-iminoesters and x-iminoketones, and the reductive amination of x-ketoesters' JOURNAL OF ORGANOMETALLIC CHEMISTRY Bd. 593-594, 15 Januar 2000, Seiten 454 - 457
- B. R. JAMES: 'Synthesis of chiral amines catalyzed homogeneously by metal complexes' CATALYSIS TODAY Bd. 37, 1997, Seiten 209 - 221
- V.I. TARAROV ET AL.: 'Approaching Highly Enantioselective Reductive Amination' SYNLETT Nr. 2, 2005, Seiten 203 - 211

## Beschreibung

Die Erfindung beschreibt die Herstellung von Aminen durch die Reaktion von Aldehyden oder Ketonen mit Ammoniak bzw. sekundären Aminen in Gegenwart von Wasserstoff und in Anwesenheit homogener Metallkatalysatoren unter milden Bedingungen. Als Metallkatalysatoren werden Komplexe später Übergangsmetalle mit phosphorhaltigen Liganden eingesetzt. Das erfindungsgemäße Verfahren ermöglicht auch die Synthese von enantiomerenreinen oder enantiomerenangereicherten Aminen durch enantioselektive bzw. diastereoselektive Reaktionsführung.

Racemische und enantiomerenreine Amine spielen eine dominierende Rolle in zahlreichen komplexen Naturstoffen wie beispielsweise den Alkaloiden, Vitaminen oder Aminosäuren, deren chemische, pharmazeutische und industrielle Bedeutung unbestritten ist. Als chemische Zwischenprodukte finden Amine u.a. Anwendung in der Synthese von Pharmazeutika, Agrochemikalien, Nahrungsmittelzusatzstoffen, Farbstoffen oder Kosmetika. Für den Bereich der Wirkstoffe spielen dabei Aminosäuren und Aminoalkohole eine überragende Rolle.

Für die Synthese von unfunktionalisierten und funktionalisierten Aminen spielt die heterogen katalysierte Aminierung von Ketonen und Aldehyden eine große Rolle (Catalytic Hydrogenation over Platinum Metals, Academic Press, New York, 1967, S. 291 ff; Catalytic Hydrogenation in Organic Synthesis, Academic Press, New York, 1979, 165 ff). Als heterogene Katalysatoren wurden beispielsweise CuCr₂O₄·CuO (Kurc et al. Chem. Prum. 1987, 37, 26), Re oder Cu (DE-A-19631521, Raney-Nickel (EP-A-0011401), Ru geträgert auf MgO/Al₂O₃ (DE-A-4010252), Ru geträgert auf γ-Al₂O₃ (EP-A-0449089), Cu geträgert auf Al₂O₃ (Barrault et al. Rev. Fr. Corps Gras 1991, 38, 103) oder Fe (CA-A-0907059) eingesetzt.

Prinzipiell hat die heterogene Reaktionsführung jedoch beträchtliche Nachteile (J. Hagen, Technische Katalyse, VCH, Weinheim, 1996, S. 10). Es hat sich gezeigt, daß charakteristische Probleme beim Stoffübergang zwischen den Phasen auftreten und im Ergebnis die Reaktionsgeschwindigkeit beträchtlich herabsetzen. Aus diesem Grund sind für die heterogen katalysierte Aminierung meist hohe Reaktionstemperaturen von bis zu 150 °C und Drucke bis zu 250 bar erforderlich.
Dies stellt einen beträchtlichen ökonomischen Aufwand beim Bau und Betrieb entsprechender Anlagen dar.
Die Entwicklung neuer Katalysatoren, welche die gewünschte Reaktion unter milderen Bedingungen ermöglichen ist deshalb von außerordentlichem Interesse. Darüber hinaus ist die Toleranz gegenüber weiteren funktionellen Gruppen, welche üblicherweise z.B. bei der Synthese von Wirkstoffen im Molekül vorhanden sind, aufgrund der drastischen Reaktionsbedingungen deutlich eingeschränkt.
Weiterhin lassen sich die heterogenen Katalysatoren nur schwierig charakterisieren, ein Fakt der die Reproduzierbarkeit der Katalyseergebnisse nachhaltig beeinträchtigen kann und das rationale Katalysatordesign bzw. Modifizierungen für spezielle Aufgabenstellung erschwert oder gar unmöglich macht.
In der Literatur sind nur sehr wenige Beispiele mit Katalysatoren in homogenen Systemen bekannt: mit Dimethylglyoximat-Komplexen des Cobalts und Rhodiums (M.V. Klyuev, M.L. Khidekel, Transition Met. Chem.,1980, 5 134-139). Zur Aktivierung der Katalysatoren müssen nahezu stöchiometrische Mengen von Natriumborhydrid eingesetzt werden. Weiterhin sind Rh- und Co-Carbonyl Komplexe (L. Marko, J. Bakos Journal of Organometallic Chemistry, 1974, 81, 411-414) und Cobalt-Cyano- Komplexe (M. Murakami, J.-W. Kang Bull. Chem. Soc. Japan, 1963, 36, 763-768) beschrieben. Aufgrund der hohen Katalysatormengen und der benötigten drastischen Bedingungen sind die beschriebenen Verfahren jedoch nicht praktikabel.
Die Patentschrift JP 11-343269 beschreibt die Synthese von Octylamin aus Octanal und Ammoniak, bei der eine Reihe von homogenen Katalysatoren wie: Eisen(II)-Sulfid, Nickelacetylacetonat,Rhodiumcarbonylacetylacetonat,
Palladiumacetylacetonat, Osmiumdodecacarbonyl(III), Indiumhexacarbonyl(VI), Platindichlor-1,5-cyclooctadien und Rutheniumacetylacetonat mit 2,2'-Bipyridyl in einem äußerst kompliziertern Verfahren bei hohen Temperatur von 150 °C eingesetzt werden. Auch die sog. Hydroaminomethylierung (P. Erbracht u.a. Chem. Rev. 1999,99, 3329-3364) - für welche eine reduktive Aminierung eines Intermediates postuliert aber bis dato nicht belegt ist - läuft unter drastischen Reaktionsbedingungen.

Aufgabe der vorliegenden Erfindung war es daher, ein Verfahren zu finden, mit welchern die Aminierung unter milden Bedingungen ermöglicht wird und die oben aufgeführten Probleme vermieden werden können.
Darüber hinaus soll das Verfahren auch die Synthese von enantiomerenreinen oder enantiomerenangereicherten Aminen durch die Verwendung von chiral modifizierten Katalysatoren erlauben.

Es wurde nun überraschenderweise gefunden, daß die gewünschten Amine sehr effizient durch die reduktive Aminierung von Ketonen und Aldehyden in Gegenwart von katalytisch wirkenden Übergangsmetallkomplexen auf der Basis phosphorhaltiger Liganden unter sehr milden Bedingungen zugänglich sind.

Unter diesen milden Reaktionsbedingungen ist bei Verwendung von chiralen Liganden eine enantioselektive Reaktionsführung möglich.

Die verwendeten Übergangsmetallkatalysatoren liefern in der reduktiven Aminierung gute bis sehr gute Ausbeuten an gewünschtem Amin. Gleichzeitig kann ein sehr hohes Amin/Alkohol-Verhältnis in den Produkten realisiert werden.

Mit dem erfindungsgemäßen Verfahren werden die bekannten Nachteile der bisher beschriebenen metallkatalysierten reduktiven Aminierung umgangen.

Gegenstand der vorliegenden Erfindung ist somit ein Verfahren zur Herstellung von Aminen der Formel (III) 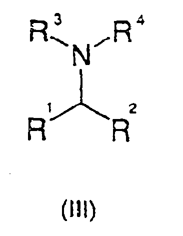 durch Umsetzung einer Verbindung der Formel (II) 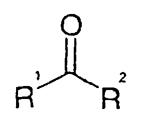 mit Ammoniak oder einem sekundären Amin der Formel (II) 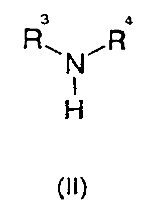 worin die Reste R¹ bis R⁴ unabhängig voneinander aus der Gruppe von Wasserstoff, (C₁-C₂₄)-Alkyl, (C₂-C₂₄)-Alkenyl, (C₂-C₂₄)-Alkinyl, (C₆-C₁₀)-Aryl, CF₃, CN, COOH, COOM, wobei M ein Kation darstellt, CHO, SO₃H, COO-Alkyl-(C₁-C₈), CONH₂, CONHAlkyl-(C₁-C₈), CONAlkyl₂-(C₁-C₈), CO-Alkyl-(C₁-C₈), CO-Phenyl, COO-Phenyl, COO-Aryl-(C₆-C₁₀), CO-Aryl-(C₆-C₁₀), P(Aryl)₂, PAlkyl₂-(C₁-C₈), PO(Aryl)₂, POAlkyl₂-(C₁-C₄), PO₃H₂, POAlkyl-(C₁-C₄)(O-Alkyl-(C₁-C₆)), PO(O-Alkyl-(C₁-C₆))₂, SO₃-Alkyl-(C₁-C₄), SO₂-Alkyl-(C₁-C₆), SO-Alkyl-(C₁-C₆) oder Si(Alkyl)₃-(C₁-C₈), und/oder R³ und R⁴ unabhängig voneinander aus der Gruppe O-Alkyl-(C₁-C₈), OCO-Alkyl-(C₁-C₈), O-Aryl, Fluor, OH, NH₂, NH-Alkyl-(C₁-C₈), N-Alkyl₂-(C₁-C₈), NHCO-Alkyl-(C₁-C₄), NHAryl-(C₆-C₁₀), NHCOO-Alkyl-(C₁-C₄), ausgewählt werden, wobei R³, R⁴ gleichzeitig Wasserstoff ist oder R³, R⁴ nicht Wasserstoff ist, wobei Alkyl im Sinne der vorliegenden Erfindung für einen nichtverzweigten oder verzweigten Rest, steht, Alkenyl für einen olefinischen Kohlenwasserstoff, Alkinyl für einen Acetylenkohlenwassersoff und Aryl für einen aromatischen Rest welcher auch ein Aromat mit mit 1-4 Heteroatomen sein kann ausgewählt aus der Gruppe N-, S- oder O-Atom. Sowohl Alkyl, Alkenyl, Alkinyl als auch Aryl können Substituenten die unabhängig voneinander Wassertoff, O-Alkyl-(C₁-C₈), OCO-Alkyl-(C₁-C₈), O-Phenyl, Phenyl, Aryl (C₆-C₁₀), Fluor, Chlor, Brom, Iod, OH, CF₃, CN, COOH, COOM, wobei M ein Kation (Li⁺, Na⁺, K⁺, Mg²⁺, Ca²⁺, NH₄⁺, N(C₁-C₁₀-Alkyl)₄⁺, N(C₁-C₁₀-Alkyl/C₆-C₁₀-Aryl)₄⁺) darstellt, CHO, SO₃H, NH₂, NH-Alkyl-(C₁-C₈), N-Alkyl₂-(C₁-C₈), NHCO-Alkyl-(C₁-C₄), COO-Alkyl-(C₁-C₈), CONH₂, CO-Alkyl-(C₁-C₈), NHCOH, NHCOO-Alkyl-(C₁-C₄), CO-Phenyl, COO-Phenyl, COO-Aryl-(C₆-C₁₀), CO-Aryl-(C₆-C₁₀), CHCH-CO₂-Alkyl-(C₁-C₈), P(Aryl)₂, CHCHCO₂H, P-Alkyl₂-(C₁-C₈), PO-Aryl₂, POAlkyl₂-(C₁-C₄), PO₃H₂, POAlkyl-(C₁-C₄)(O-Alkyl-(C₁-C₆)), PO(O-Alkyl-(C₁-C₆))₂, SO₃-Alkyl-(C₁-C₄), SO₂-Alkyl-(C₁-C₆), SO-Alkyl-(C₁-C₆) oder Si(Alkyl)₃-(C₁-C₈) bedeuten, tragen.
R³ und R⁴ können durch kovalente Bindungen verknüpft sein, so daß R³ und R⁴ jeweils einen vier- bis achtgliedrigen Ring bilden.

Die Umsetzung erfolgt in Gegenwart von Wasserstoff und einem homogenen Katalysatorsystem enthaltend mindestens ein Metallatom ausgewählt aus der Gruppe Rh, und einen oder mehrere bidentate achirale oder chirale Liganden
wobei als Ligand 1,4-Bis(diphenylphosphino)butan), 1,4-Bis(dicyclohexylphosphino)butan, 2-Diphenylphosphinomethyl-4-diphenylphosphino-1-tert-butoxycarbonylpyrrolidin, 2,3-O-isopropyliden-2,3-dihydroxy-1,4-bis(diphenylphosphino)butane, (2*R*,3*R*,5*R*,6*R*)-2,3-Dimethoxy-2,3-dimethyl-5,6-bis(diphenylphosphinomethyl)-1,4-dioxan, 2,2'-Bis[[bis(3-sulfophenyl)phosphino]methyl]- 4,4',7,7'-tetrasulfo-1,1'-binapthyl Octanatriumsalz, Diphosphinit-Liganden auf Kohlenhydratbasis, 1,2-Bis(diphenylphosphinoxy)ethan), (1*R*,2*R*)-(trans)-1,2-Bis-(diphenylphosphinoxy)cyclohexan, (2*R*)-1-[[(diphenylphosphino)(cyclopenthyl)-amino]methyl]-2-diphenylphosphinoxy-3-(1-naphthalenyloxy)propan und/oder (4*S*)-2-(2-(Diphenylphosphino)phenyl)-4-isopropyl-1,3-oxazoline verwendet werden und wobei als Lösungsmittel Alkohole, Wasser und Gemische derselben eingesetzt werden.

In einer bevorzugten Ausführung bedeutet R¹ bis R⁴ unabhängig voneinander Wasserstoff, (C₁-C₁₂)-Alkyl, (C₂-C₁₂)-Alkenyl, (C₂-C₁₂)-Alkinyl, (C₆-C₁₀)-Aryl, CF₃, CN, COOH, COOM, wobei M ein Kation (Li⁺, Na⁺, K⁺, Mg²⁺, Ca²⁺, NH₄⁺, N(C₁-C₁₀-Alkyl)₄⁺, N(C₁-C₁₀-Alkyl/C₆-C₁₀-Aryl)₄⁺)darstellt, COO-Alkyl-(C₁-C₈), CONH₂, CO-Alkyl-(C₁-C₈), CO-Phenyl, COO-Phenyl, COO-Aryl-(C₆-C₁₀), CO-Aryl-(C₆-C₁₀), PO(Aryl-C₆-C₁₀)₂, POAlkyl₂-(C₁-C₄), PO₃H₂, PO(Alkyl-(C₁-C₄))(OAlkyl-(C₁-C₄)), PO(O-Alkyl-(C₁-C₆))₂ oder Si(Alkyl)₃-(C₁-C₈) und/oder R³ und R⁴ unabhängig voneinander aus der Gruppe O-Alkyl-(C₁-C₈), OCO-Alkyl-(C₁-C₈), O-Aryl (C₆-C₁₀), OH, NH₂, NH-Alkyl-(C₁-C₈), N-Alkyl₂-(C₁-C₈), NHCO-Alkyl-(C₁-C₄), NHCOO-Alkyl-(C₁-C₄), ausgewählt werden,
wobei Alkyl einen nichtverzweigten oder verzweigten Rest, Alkenyl einen olefinischen Kohlenwasserstoff, Alkinyl einen Acetylenkohlenwassersoff bedeuten und Aryl einen aromatischen Rest welcher auch ein Aromat mit 1-4 Heteroatomen ausgewählt aus der Gruppe N-, S- oder O-Atom sein kann. Sowohl Alkyl, Alkenyl und Alkinyl als auch Aryl können Substituenten die unabhängig voneinander Wassertoff, O-Alkyl-(C₁-C₈), OCO-Alkyl-(C₁-C₈), O-Phenyl, Phenyl, Aryl- C₆-C₁₀, Fluor, Chlor, Brom, lod, OH, SiAlkyl₃-(C₁-C₈), CF₃, CN, COOH, COOM, wobei M ein einwertiges Kation ausgewählt ist aus der Gruppe Na, K, Rb, Cs, NH₄, N(C₁-C₁₀-Alkyl)₄, N(C₁-C₁₀-Alkyl/ C₆-C₁₀-Aryl)₄ darstellt, SO₃H, N-Alkyl₂-(C₁-C₈), SO₂-Alkyl-(C₁-C₆), SO-Alkyl-(C₁-C₆), NHCO-Alkyl-(C₁-C₄), COO-Alkyl-(C₁-C₈), CONH₂, CO-Alkyl-(C₁-C₈), CO-Phenyl, COO-Phenyl, COO-Aryl-(C₆-C₁₀), CO-Aryl-(C₆-C₁₀), PO-Phenyl₂, POAlkyl₂-(C₁-C₄), PO₃H₂, POAlkyl-(C₁-C₄)(O-Alkyl-(C₁-C₆)), PO(O-Alkyl-(C₁-C₆))₂, Si(Alkyl)₃-(C₁-C₈), wobei Alkyl und Aryl oben genannte Bedeutung haben. R³ und R⁴ können durch kovalente Bindungen verknüpft sein, so daß ein fünf- bis siebengliedriger Ring vorliegt.

Als homogener Metallatom-Ligand-Komplex werden Metallkomplexe angewandt, die als Zentralatom Rhodium enthalten.

Typische Vertreter der im erfindungsgemäßen Verfahren verwendeten Ligandsysteme sind dppb (1,4-Bis(diphenylphosphino)butan), dcypb (1,4-Bis(dicyclohexylphosphino)butan), bppm (2-Diphenylphosphinomethyl-4-diphenylphosphino-1-tert-butoxycarbonylpyrrolidin), diop (2,3-O-isopropyliden-2,3-dihydroxy-1,4-bis(diphenylphosphino)butan) (Kagan et al. J. Amer. Chem. Soc. (1972), 94, 6429), (2*R*,3*R*,5*R*,6*R*)-2,3-Dimethoxy-2,3-dimethyl-5,6-bis(diphenylphosphinomethyl)-1,4-dioxan (Berens et al. J. Org. Chem. (1995), 60, 8204), BINAS (2,2'-Bis[[bis(3-sulfophenyl)phosphino]methyl]- 4,4',7,7'-tetrasulfo-1,1'-binapthyl Octanatriumsalz) (Herrmann et al. Inorg. Synth. (1998), 32, 8), Diphosphinit-Liganden auf Kohlenhydratbasis wie z.B. in DD 140036 und WO 95/18787 beschrieben und dpoe (1,2-Bis(diphenylphosphinoxy)ethan),bdpch ((1*R*,2*R*)-(trans)-1,2-Bis-(diphenylphosphinoxy)cyclohexan) PROPRAHOS-Analog (2*R)*-*1*-[[(diphenylphosphino)(cyclopenthyl)amino]methyl]-2-diphenylphosphinoxy-3-(1-naphthalenyloxy)propan (Krause et al. J. Mol. Catal. A: Chem. (1995), 104, 147), (4*S*)-2-(2-(Diphenylphosphino)phenyl)-4-isopropyl-1,3-oxazoline (Koch G., Lloyd-Jones G. C., Loiseleur O., Pfaltz A., Pretot R., Schaffner S., Schnider P., von Matt P. Recl. Trav. Chim. Pays-Bas 1995, 114, 206-10).

Die phosphorhaltigen Liganden lassen sich unter den dem Fachmann geläufigen Bedingungen herstellen (beispielsweise nach Methoden wie sie in Chemistry of Organophosphorous Compounds, Ed. F. R. Hartley, Serial Ed. S. Patai, Vol. 1, John Whiley, 1990 beschrieben sind). Die Liganden und oder Metallkomplexe sind teilweise auch kommerziell erhältlich (beispielsweise von Aldrich oder Strem/ABCR). Die Metallkomplexe lassen sich beispielsweise synthetisieren, indem man in bekannter Weise (EP-A -0158875; EP-A-0437690) durch Umsetzung mit Rhodium-, komplexen, die labile Liganden enthalten (z.B. [Rh(COD)₂]BF₄) mit den phosphorhaltigen Liganden katalytisch aktive Komplexe generiert. Darüberhinaus können alle, dem Metallorganiker geläufigen Methoden zur Generierung entsprechender Komplexe genutzt werden.
Die Katalysatoren können dabei in situ aus dem Metallprecursor und dem Liganden erzeugt werden, oder sie werden in isolierter Form eingesetzt.

Das erfindungsgemäße Verfahren wird in der Regel bei einer Temperatur von -40 - 100 °C, bevorzugt bei -20 - 60 °C durchgeführt.

Der Wasserstoffanfangsdruck kann in einem großen Bereich zwischen 0.1 bar und 300 bar für das erfindungsgemäße Verfahren variiert werden. Bevorzugt arbeitet man bei 1 bar -100 bar. Besonders bevorzugt wird zwischen 20 und 60 bar gearbeitet

Für das erfindungsgemäße Verfahren kann es Vorteilhaft sein, in Gegenwart von Additiven zu Arbeiten.

Additive sind Säuren, wie beispielsweise p-Toluolsulfonsäure, Tetrafluorborsäure, Phosphorsäure, Schwefelsäure, Essigsäure, Basen wie Natronlauge, Kalilauge, tertiäre Amine, Protoneneschwamm, Cäsiumcarbonat, Acetat, Soda, Salze wie die Halogenide der Alkalimetalle oder Halogenide von Ammoniumsalzen, Phasen-Transfer Katalysatoren, Tenside, Cyclodextrine, die von 0 - 100 mol-% bezogen auf das eingesetzte Amin (II) angewandt werden.

Lösungsmittel für die reduktive Aminierung sind Alkohole, insbesondere C1-C6-Alkanole, insbesondere bevorzugt Methanol, Ethanol, Propanol, Isopropanol oder aber auch Wasser und Gemische derselben.
Das Verfahren kann auch in einen 2-Phasen-System wie beispielsweise in DE 19737053 beschrieben durchgeführt werden.

Der Katalysator wird üblicherweise in Mengen von 0.001 bis 5 mol-%, bevorzugt 0.001 bis 0.01 mol-%, bezogen auf die Carbonylkomponente der Formel (I) eingesetzt.

Die nachstehenden Beispiele dienen der Erläuterung der Erfindung, ohne diese darauf zu beschränken.

### Beispiel 1

In einem Autoklav wurde eine Lösung von 5.0 mmol Acetophenon, 5.0 mmol Piperidin und 0.01 mmol Rh[(dppb)(COD)]BF₄ in 10 ml Methanol bei Raumtemperatur und einem Anfangswasserstoffdruck von 51-52 bar 19.7 Stdn. lang gerührt. Unter diesen Bedingungen wurde das Keton zu 25.4 % umgesetzt. Das ¹H-NMR-spektroskopisch bestimmte Verhältnis von 1-N-Piperidinylethylbenzol zu 1-Phenylethylcarbinol im Produkt betrug 1/10 (vgl. Tabelle 1).

### Beispiel 2

In einem Autoklav wurde eine Lösung von 5.0 mmol Acetophenon, 5.0 mmol Piperidin, 0.2 mmol p-Toluolsulfonsäure und 0.01 mmol Rh[(dppb)(COD)]BF₄ in 10 ml Methanol bei Raumtemperatur und einem Anfangswasserstoffdruck von 51-52 bar 16 Stdn. lang gerührt. Unter diesen Bedingungen wurde das Keton zu 5.6 % umgesetzt. Das Verhältnis von 1-N-Piperidinylethylbenzol zu 1-Phenylethylcarbinol im Produkt betrug 2/1 (vgl. Tabelle 1).

### Beispiel 3 (Vergleichsbeispiel)

In einem Autoklav wurde eine Lösung von 5.0 mmol Acetophenon, 5.0 mmol Benzylamin und 0.01 mmol Rh[(dppb)(COD)]BF₄ in 10 ml Methanol bei Raumtemperatur und einem Anfangswasserstoffdruck von 51-52 bar 20 Stdn. lang gerührt. Unter diesen Bedingungen wurde das Keton zu 10.7 % umgesetzt. Das Verhältnis von 1-N-Benzylaminoethylbenzol zu 1-Phenylethylcarbinol im Produkt betrug 1/10 (vgl. Tabelle 1).

### Beispiel 4 (Vergleichsbeispiel)

In einem 300 ml Autoklav mit intensiver magnetischer Rührer wurde eine Lösung von 60 mmol Acetophenon in 40 ml Toluol, 40 ml 25 %ige wässrige Ammoniaklösung, 0.15 mmol [Ir[(COD)Cl]₂ und 6 ml einer 0.1 molaren Lösung des 2,2'-Bis[[bis(3-sulfophenyl)phosphino]methyl]- 4,4',7,7'-tetrasulfo-1,1'-binapthyl Octanatriumsalzes (BINAS) gegeben und bei 130 °C und einem Anfangswasserstoffdruck von 46 bar gerührt. Nach 13 Stunden wurde Autoklav abgekühlt und entspannt, die organische Phase über MgSO₄ getrocknet und gaschromatographisch untersucht. Nach Verdampfen des Lösungsmittel der Rückstand wurde NMR-spectroskopisch untersucht. Bei einem Umsatz von 38 % wurde das Verhältnis von 7/31 des 1-Phenylethylamines zu 1-Phenylethanol festgestellt (vgl. Tabelle 1).

### Beispiel 5 (Vergleichsbeispiel)

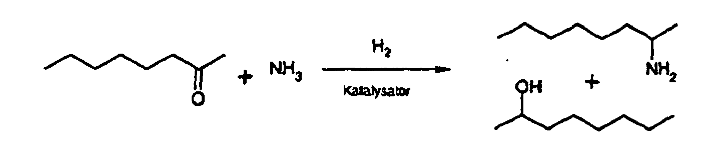

In einem 300 ml Autoklav mit intensiver magnetischer Rührung wurde eine Lösung von 60 mmol 2-Octanon in 40 ml Heptan, 40 ml 25 %ige wässrige Ammoniaklösung, 0.15 mmol [Ir[(COD)Cl]₂, 2.4 mmol teilmethylierten β-Cyclodextrin von Cyclolab (Budapest, Ungarn) und 6 ml einer 0.1 molaren Lösung des 2,2'-Bis[[bis(3-sulfophenyl)phosphino]methyl]- 4,4',7,7'-tetrasulfo-1,1'-binapthyl Octanatriumsalzes (BINAS) gegeben und bei 130 °C und einem Wasserstoffanfangsdruck von 89 bar gerührt. Nach 7 Stunden wurde Autoklav abgekühlt und entspannt, die organische Phase über MgSO₄ getrocknet und gaschromatographisch untersucht. Nach Verdampfen des Lösungsmittel der Rückstand wurde NMR-spectroskopisch untersucht. Bei einem Umsatz von 10 % wurde das Verhältnis von 7/3 des 2-Octylamines zu 2-Octanol festgestellt.

### Beispiel 6 (Vergleichsbeispiel)

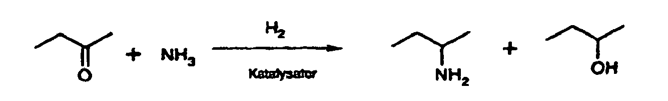

In einem 100 ml Autoklav mit intensiver magnetischer Rührung wurde 0.15 mol Butanon, 60 ml 25 %ige wässrige Ammoniaklösung, 0.15 mmol [Ir[(COD)Cl]₂ und 6 ml einer 0.1 molaren Lösung des BINAS gegeben und bei 130 °C und einem Wasserstoffanfangsdruck von 110 bar gerührt. Nach 5 Stunden wurde Autoklav abgekühlt und entspannt, die wäßrige Lösung wurde NMR-spectroskopisch untersucht. Bei einem Umsatz von 80 % wurde das Verhältnis von 1/2 des 2-Butylamines zu 2-Butanol festgestellt. Der Anteil des sekundären Amines betrug 6 %.

### Beispiel 7

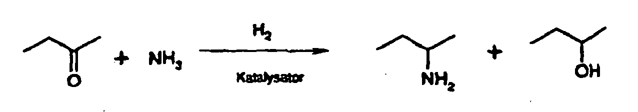

In einem 100 ml Autoklav mit intensiver magnetischer Rührer wurde 0.15 mol Butanon, 50 ml 25 %ige wässrige Ammoniaklösung, 0.15 mmol [Rh[(COD)Cl]₂ und 6 ml einer 0.1 molaren Lösung des BINAS gegeben und bei 100 °C und einem Wasserstoffanfangsdruck von 63 bar gerührt. Nach 7 Stunden wurde Autoklav abgekühlt und entspannt, die wäßrige Lösung (13 % Edukt, 14 % 2-Butanol und 73 % 2-Buthylamin) wurde mehrfach ausgeethert, die organische Phase über Ätzkali getrocknet und über eine 30-cm-Vigreux-Kolonne fraktioniert. Die Fraktion zwischen 55 und 66 °C (10.2 g) bestand zu 30 % (GC-Analyse) aus dem gewünschten 2-Butylamin.

### Beispiele 8 bis 13

Diese Beispiele werden analog zu Beispielen 1-3 durchgeführt. Aminkomponenten, Katalysatoren sowie die Reaktionsergebnisse sind in Tabelle 2 angegeben.

### Beispiel 14

In einem 100 ml Autoklav mit Tropftrichter wurden 5.1 ml (50 mmol) Benzaldehyd mit 20 ml Methanol versetzt, das bei 10 °C mit Ammoniak gesättigt worden war. Der Autoklav wurde verschlossen, 91 bar Wasserstoff aufgepresst und auf 80 °C erwärmt. Aus dem Tropftrichter wurden anschließend eine Lösung von 0.05 mmol [Rh(dcypb)]BF₄ in 10 ml Methanol zugegeben und bei 80 °C noch 2 Stunden lang gerührt. Nach Abkühlen und Verdampfen des Lösungsmittels wurde der Rückstand gaschromatographisch und NMR-spectroskopisch untersucht. Bei einem Umsatz von über 99 % wurde das Verhältnis von 25/32/43 des Benzylamines zu Dibenzylamin und zu Benzylalkohol festgestellt.

### Beispiel 15 (Vergleichsbeispiel)

0.2 ml (2 mmol) Benzaldehyd wurden in 2 ml Heptan gelöst und nach Zugabe von eine Lösung von 23 mg (0.04 mmol) Tris-(3-sulphophenyl)phosphin Trinatriumsalz (TPPTS) und 7.2 mg (0.02 mmol) [Rh[(C₈H₁₄)₂Cl]₂ in 2 ml 25 %ige wässrige Ammoniaklösung unter lebhaftern Rühren in einem Autoklav bei 46 bar und 90 °C hydriert. Nach 8 Stunden wurde Autoklav abgekühlt und entspannt, die organische Phase über MgSO₄ getrocknet und gaschromatographisch untersucht. Bei einem Umsatz von über 99 % wurde das Verhältnis von 52/20/18 des Benzylamines zu Dibenzylamin und zu Benzylalkohol festgestellt.

### Beispiel 16

Dieses Beispiel wurde analog zu Beispiel 14 durchgeführt. Die Reaktionsbedingungen und Ergebnisse sind in Tabelle 2 angegeben.

### Beispiel 17.

In einem 100 ml Autoklav mit Tropftrichter wurde eine Lösung von 5.1 ml (50 mmol) Benzaldehyd in 15 ml Ethanol mit 20 ml 25 %ger Ammoniak-Lösung versetzt. Der Autoklav wurde verschloßen, 78 bar Wasserstoff aufgepresst und auf 100 °C erwärmt. Aus dem Tropftrichter wurden anschließend eine Lösung von 0.1 mmol [Rh(dppb)]BF₄ in 10 ml Methanol zugegeben und bei 100 °C noch 2 Stunden lang gerührt. Nach Abkühlen und Verdampfen des Lösungsmittels wurde der Rückstand gaschromatographisch und NMR-spectroskopisch untersucht. Bei einem Umsatz von über 99 % wurde das Verhältnis von 15/85 des Benzylamines zu Benzylalkohol festgestellt.

### Beispiel 18.

In einem 100 ml Autoklav mit Tropftrichter wurde eine Lösung von 5.1 ml (50 mmol) Benzaldehyd in 10 ml Methanol mit 20 ml 25 %ger Ammoniak-Lösung versetzt. Der Autoklav wurde verschlossen, 69 bar Wasserstoff aufgepresst und auf 90 °C erwärmt. Aus dem Tropftrichter wurden anschließend eine Lösung von 0.05 mmol [Rh[(COD)Cl]₂ und 0.2 mmol 2,2'-Bis[[bis(3-sulfophenyl)phosphino]methyl]- 4,4',7,7'-tetrasulfo-1,1'-binapthyl Octanatriumsalz (BINAS) in 5 ml 5 %ger wässrige Ammoniaklösung zugegeben und bei 90 °C noch 1,5 Stunden lang gerührt. Nach Abkühlen und Verdampfen des Lösungsmittels wurde der Rückstand gaschromatographisch und NMR-spectroskopisch untersucht. Bei einem Umsatz von 99 % wurde das Verhältnis von 58/25/17 des Benzylamines zu Dibenzylamin und zu Benzylalkohol festgestellt.

### Beispiel 19.

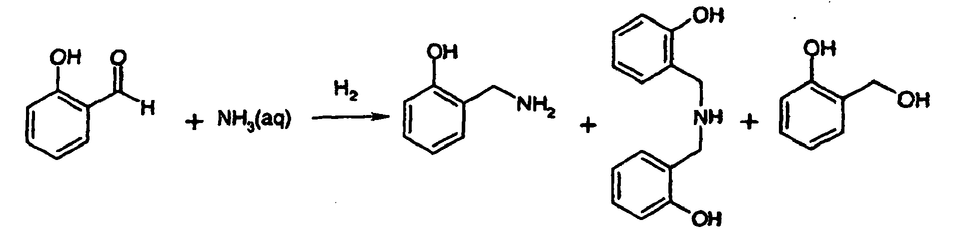

In einem 100 ml Autoklav mit Tropftrichter wurde eine Lösung von 5.3 ml (50 mmol) Salicylaldehyd in 30 ml Ethanol zu 10 ml 25 %ger Ammoniak-Lösung unter Rühren getropft. Der Autoklav wurde verschlossen, 58 bar Wasserstoff aufgepreßt und auf 90 °C erwärmt. Aus dem Tropftrichter wurde anschließend eine Lösung von 0.05 mmol [Rh[(COD)Cl]₂ und 0.2 mmol 2,2'-Bis[[bis(3-sulfophenyl)phosphino]methyl]-4,4',7,7'-tetrasulfo-1,1'-binapthyl Octanatriumsalz (BINAS) in 5 ml 5 %ger Ammoniak-Lösung zugegeben und bei 90 °C noch 10 Stunden lang gerührt. Nach Abkühlen und Verdampfen des Lösungsmittels wurde der Rückstand NMR-spectroskopisch untersucht. Bei einem Umsatz von 96 % wurde das Verhältnis von 51/45 des o-Hydroxybenzylamines zu Di(o-hydroxybenzyl)amin festgestellt.

### Beispiel 20.

In einem 30 ml Autoklav mit Tropftrichter wurde eine Lösung von 0.01 mmol Rh[(dppb)COD]BF₄ in 5 ml Methanol bei Raumtemperatur und 50 bar Wasserstoffdruck 15 min lang hydriert. Aus dem Tropftrichter wurde anschließend eine Lösung von 5 mmol 4-hydroxybenzaldehyd und 10 mmol Piperidin in 10 ml Methanol zugegeben und weitere 2 Stunden hydriert. Nach Verdampfen des Lösungsmittels wurde der Rückstand NMR-spectroskopisch untersucht. Bei einem Umsatz von über 99 % wurde das Verhältnis von 94/1 des N-(4-Hydroxybenzyl)-piperidines zu 4-Hydroxybezylalkohol festgestellt.

### Beispiele 21 bis 28

Diese Beispiele werden analog zu Beispiel 20 durchgeführt. Aldehyde sowie die Reaktionsergebnisse sind in Tabelle 3 angegeben.

### Beispiele 29 bis 34

Diese Beispiele werden analog zu Beispiel 1 durchgeführt. Katalysatoren sowie die Reaktionsergebnisse sind in Tabelle 4 angegeben.

### Beispiel 35.

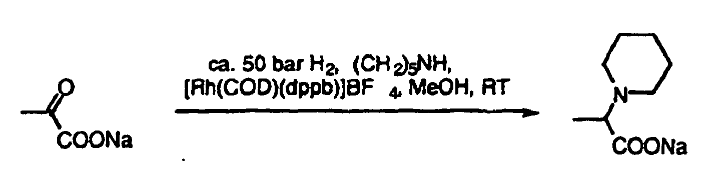

In einem Autoklav wurde ein Lösung von 5.0 mmol Natriumpyruvat, 5.0 mmol Piperidin und 0.01 mmol Rh[(dppb)COD]BF₄ in 10 ml MeOH bei Raumtemperatur und einem Anfangswasserstoffdruck von 51-52 bar 89 Stdn. lang gerührt. Unter diesen Bedingungen wurde die Ketosäure zu 82 % umgesetzt. Das ¹H-NMR-spektroskopisch bestimmte Verhältnis von Natrium-N,N-Pentamethylenalaninat zu Natrium-lactat im Produkt betrug 9.2/1.

### Beispiel 36.

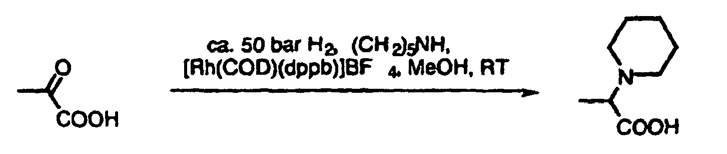

In einem Autoklaven wurde ein Lösung von 5.0 mmol Brenztraubensäure, 5.0 mmol Piperidin und 0.01 mmol Rh[(dppb)COD]BF₄ in 10 ml MeOH bei Raumtemperatur und einem Anfangswasserstoffdruck von 51-52 bar 20 Stdn. lang gerührt. Unter diesen Bedingungen wurde die Ketosäure zu 99.6 % umgesetzt. Das ¹H-NMR-spektroskopisch bestimmte Verhältnis von N,N-Pentamethylenalanin zu Milchsäure im Produkt betrug 1.4/1.

### Beispiel 37. (Vergleichsbeispiel)

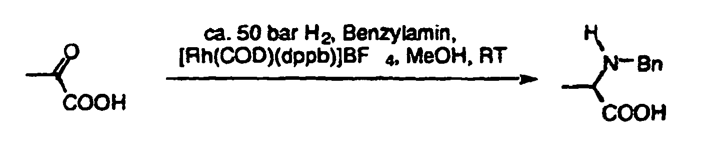

In einem Autoklaven wurde ein Lösung von 5.0 mmol Brenztraubensäure, 5.0 mmol Benzylamin und 0.01 mmol Rh[(dppb)COD]BF₄ in 10 ml MeOH bei Raumtemperatur und einem Anfangswasserstoffdruck von 51-52 bar 20 Stdn lang gerührt. Unter diesen Bedingungen wurde die Ketosäure zu 94 % umgesetzt. N-Benzylalanin ist unlöslich in der Reaktionsmischung und konnte durch Filtration abgetrennt werden. Nach Waschen mit MeOH und Ether wurde das reine Produkt erhalten (Fp. 238-239 °C).

### Beispiel 38.

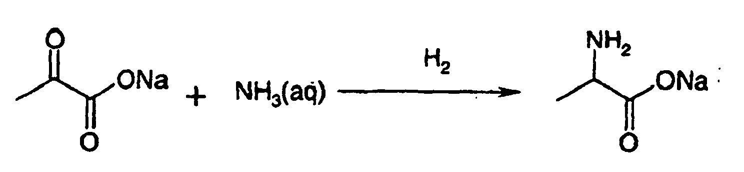

In einem 100 ml Autoklav mit Tropftrichter wurde eine Lösung von 3.3 g (30 mmol) Brenztraubensäure Natriumsalz und 40 ml 25 %ger Ammoniak-Lösung gegeben. Der Autoklav wurde verschlossen, 33 bar Wasserstoff aufgepreßt und auf 60 °C erwärmt. Aus dem Tropftrichter wurde anschließend eine Lösung von 0.15 mmol [Rh[(COD)Cl]₂ und 0.6 mmol 2,2'-Bis[[bis(3-sulfophenyl)phosphino]methyl]- 4,4',7,7'-tetrasulfo-1,1'-binapthyl Octanatriumsalz (BINAS) in 10 ml 25 %ger Ammoniak-Lösung zugegeben und bei 60 °C 16 Stunden lang gerührt. Nach Abkühlen und Entspannen des Autoklaves der überschüssige Ammoniak wurde im Vakuum abgezogen, mit 10 %ige Salzsäure gegen Bromthymolblau neutralisiert, auf eine lonaustauschersäule gegeben (Dowex AG 50W-X8, H-Form, 200-400 mesh 25 X 2 cm), mit 100 ml Wasser gewaschen und mit 5 % Ammoniakwasser eluirt. Nach Eindampfen im Vakuum. wurde 2.0 g (75 %) Alanin als farblose kristalline Rückstand erhalten.

### Beispiel 39

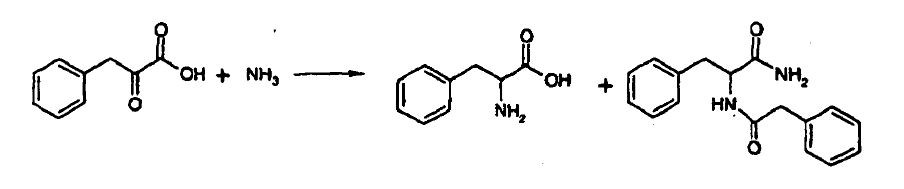

In einem 300 ml Autoklav mit intensiver magnetischer Rührer wurde eine Lösung von 5 g (30 mmol) Phenylbrenztraubensäure in 50 ml Ethanol, 20 ml 25 %ige wässrige Ammoniaklösung, 0.15 mmol [Rh[(COD)Cl]₂ und 6 ml einer 0.1 molaren Lösung des 2,2'-Bis[[bis(3-sulfophenyl)phosphino]methyl]- 4,4',7,7'-tetrasulfo-1,1'-binapthyl Octanatriumsalzes (BINAS) gegeben und bei 60 °C und einem Anfangswasserstoffdruck von 42 bar gerührt. Nach 24 Stunden wurde Autoklav abgekühlt und entspannt. N-Phenylacetyl-phenylalanin-amid ist unlöslich in der Reaktionsmischung und konnte durch Filtration abgetrennt werden. Nach waschen mit Wasser und Alkohol wurde 1.8 g (43 %) des reinen N-Phenylacetyl-phenylalaninamides erhalten. Aus der Mutterlauge wurde das überschüssige Ammoniak im Vakuum entfernt. Die wässrige Lösung wurde mit 10 %ige Salzsäure gegen Bromthymolblau neutralisiert und auf eine Säule mit Dowex (AG 50W-X8, H-Form, 200-400 mesh 25 X 2 cm) aufgespult, mit 100 ml Wasser gewaschen und mit 5 % Ammoniakwasser eluirt. Nach Eindampfen im Vakuum. wurde 0.72 g (15 %) Phenylalanin als farblose kristalline Rückstand erhalten.

### Beispiel 40 (Vergleichsbeispiel)

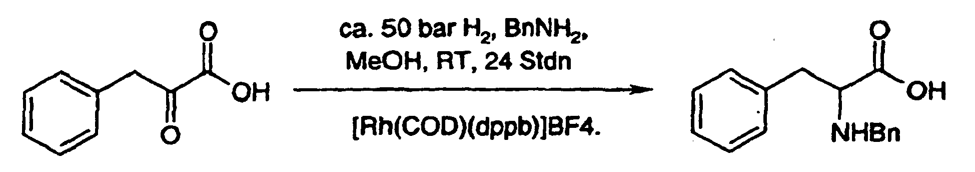

In einem Autoklaven wurde ein Lösung von 5.0 mmol Phenylbrenztraubensäurer, 5.0 mmol Benzylamin und 0.01 mmol Rh[(dppb)(COD)]BF₄ in 10 ml MeOH bei Raumtemperatur und einem Anfangswasserstoffdruck von 52 bar 20 Stdn lang gerührt. Unter diesen Bedingungen wurde die Ketosäure zu 99 % umgesetzt. N-Benzylphenylalanin ist unlöslich in der Reaktionsmischung und konnte durch Filtration abgetrennt werden. Nach Waschen mit MeOH und Ether wurde das reine N-Benzyl-phenylalanin erhalten (Fp. 219-220 °C). Ausbeute 0.90 g (71 %).

### Beispiele 41 bis 44 (Vergleichsbeispiele)

Diese Beispiele werden analog zu Beispiel 40 durchgeführt. Chirale Liganden sowie die Ausbeuten und Enantioselektivitäten sind in Tabelle 5 angegeben.

**Tabelle 1. Übersicht über die reduktive Aminierung von Acetophenon**

| | | | | | |
|---|---|---|---|---|---|
| 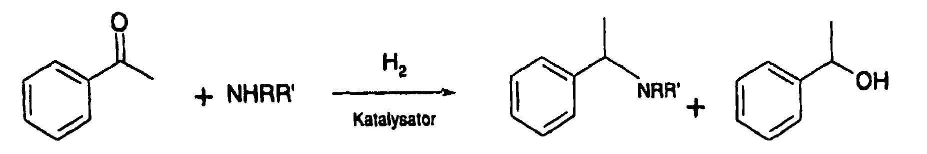 | | | | | |

| **Beispiel** | **Katalysator** | **Amin (Acetophenon:Amin)** | **Zeit (Stdn.)** | **Umsatz von RR'C=O (%)** | **C-N/C-OH Verhältnis** |
|---|---|---|---|---|---|
| 1^{a} | Rh[(dppb)COD]BF₄^{b} | (CH₂)₅NH (1:1) | 19.7 | 25.4 | 0.1 |
| 2^{a} | Rh[(dppb)COD]BF₄^{b} | (CH₂)₅NH (1:1)^{c} | 16 | 5.6 | 2 |
| 3^{a}* | Rh[(dppb)COD]BF₄^{b} | BnNH₂ (1:1) | 20 | 10.7 | 0.1 |
| 4^{d}* | [Ir(COD)Cl]₂/BINAS^{e} (1:4) | NH₃ (1:10) | 13 | 38 | 0.22 |

| | | | | | |
|---|---|---|---|---|---|
| ^{a} Bedingungen: RT, 51-52 bar (Anfangsdruck), 5.0 mmol Acetophenon, 5.0 mmol Amin, 0.01 mmol Präkatalysator, 10 ml MeOH, ; ^{b} dppb = 1,4-Bis(diphenylphosphino)butan; ^{c}TsOH als Additiv (molares Verhältnis Additiv:Kat.=20:1) ^{d}Bedingungen: 130 °C, 46 bar (Anfangsdruck), 60 mmol Acetophenon, 40 ml 25 %ger Wässrige Ammoniaklösung, 0.01 mmol Präkatalysator, 40 ml Toluol; ^{e} BINAS = 2,2'-Bis[[bis(3-sulfophenyl)phosphino]methyl]-4,4',7,7'-tetrasulfo-1,1'-binapthyl Octanatriumsalz. * Vergleichsbeispiel | | | | | |

**Tabelle 2. Reduktive Aminierung von Benzaldehyd**

| | | | | | | | | |
|---|---|---|---|---|---|---|---|---|
| 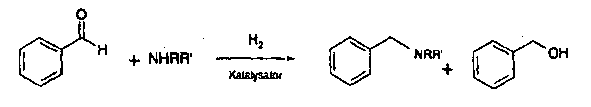 | | | | | | | | |

| Beispiel | Katalysator | Amin (Sub./Amin) | Lösungsmittel | Additiv | Temp./ Druck | Zeit (Stdn.) | Umsatz von RR'C=O (%) | C-N(prim:sec) /C-OH |
|---|---|---|---|---|---|---|---|---|
| 8 | Rh(PPh₃)₃Cl | (CH₂)₅NH(1 :1) | MeOH | - | RT/52 | 20 | 94.5 | 0.13 |
| 9 | [Rh(dppb)(COD)]BF₄^{b} | (CH₂)₅NH(1:1) | MeOH | - | RT/52 | 20 | > 99.9 | 1.5 |
| 10 | [Rh(dppb)(COD)]BF₄^{b} | (CH₂)₅NH(1:1) | MeOH | TsOH^{a} | RT/52 | 20 | > 99.9 | 1.3 |
| 11 | [Rh(DPOE)(COD)]BF₄^{c} | (CH₂)₅NH(1:1) | MeOH | - | RT/52 | 20 | > 99.9 | 1.8 |
| 12 | [Rh(DPOE)(COD)]BF₄^{c} | (CH₂)₅NH(1:1) | MeOH | TsOH^{a} | RT/52 | 20 | > 99.9 | 1.4 |
| 13* | [Rh(dppb)(COD)]BF₄^{b} | BnNH₂(1:1) | MeOH | - | RT/52 | 20 | 38.7 | nur Amin |
| 14 | [Rh(dcypb)(COD)]BF₄^{g} | NH₃(1:6) | MeOH | - | 80/100 | 2 | > 99 | 1.4 (5:6) |
| 15* | [Rh(COD)Cl₂]₂/TPPTS^{d}(1:4) | NH₃(1:7) | Hept./H₂O | - | 90/46 | 8 | > 99 | 4 (5:2) |
| 16* | [Rh(COD)Cl₂]₂/BINAS^{a}(1:4) | NH₃(1:7) | Tol./H₂O | BnNMe₃Cl^{t} | 100/120 | 3 | > 99 | 1 (5:1) |
| 17 | [Rh(dppb)(COD)]BF₄^{b} | NH₃(1:6) | EtOH/H₂O | - | 100/79 | 2 | > 99 | 0.2(1:0) |
| 18 | [Rh(COD)Cl₂]₂/BINAS^{e}(1:4) | NH₃(1:8) | MeOH/H₂O | - | 90/69 | 1.5 | > 99 | 5 (12:5) |

| | | | | | | | | |
|---|---|---|---|---|---|---|---|---|
| ^{a}Molares Verhältnis von TsOH:Kat.=20:1; ^{b}dppb = 1,4-Bis(diphenylphosphino)butan; ^{c}DPOE = = 1,2-Bis(diphenylphosphinoxy)ethan; ^{d}TPPTS = Tris-(3-sulphophenyl)phosphin Trinatriumsalz; ^{e}BINAS = 2,2'-Bis[[bis(3-sulfophenyl)phosphino]methyl]-4,4',7,7'-tetrasulfo-1,1'-binapthyl Octanatriumsalz; ^{f}molares Verhältnis von BnNMe₃Cl:Kat.= 10:1; ^{g}dcypb = 1,4-Bis(dicyclohexylphosphino)butan. * Vergleichsbeispiel | | | | | | | | |

**Tabelle 3. Reduktive Aminierung von Aldehyden mit Piperidin ^{a}**

| | | |
|---|---|---|
| 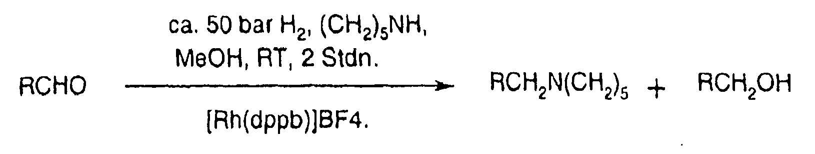 | | |

| **Beispiel** | **Aldehyd** | **C-N/C-OH** |
|---|---|---|
| 20 | 4-HOC₆H₄CHO | 94 |
| 21 | 2-MeC₆H₄CHO | 50 |
| 22 | 4-MeOC₆H₄CHO | 12 |
| 23 | C₈H₄CHO | 8.6 |
| 24 | 4-ClC₆H₄CHO | 6.7^{b} |
| *25 | 4-NO₂C₆H₄CHO | ^{c} |
| 26 | PhCHMeCHO | 7.5 |
| 27 | EtCHMeCHO | 18 |
| 28 | *n*-C₇H₁₅CHO | 210 |

| | | |
|---|---|---|
| ^{a}Bedingungen siehe Beispiel 20; ^{b}ca. 40 % 4-ClC₆H₄CH(OMe)₂; ^{c}kein Reduktionsprodukt, ca. 40 % 4-NO₂C₆H₄CH(OMₑ)₂; * Vergleichsbeispiel | | |

**Tabelle 4. Reduktive Aminierung von 2-Phenylpropanal^{a}**

| | | | | | |
|---|---|---|---|---|---|
| 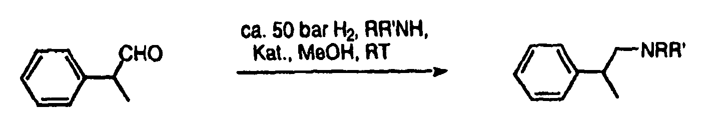 | | | | | |

| **Beispiel** | **Katalysator** | **Amin (Amin:C=O)** | **Additiv (Ad:Kat)** | **Umsatz von RR'C=O (%)^{b}** | **C-N/C-OH** |
|---|---|---|---|---|---|
| 29* | Rh(PPh₃)₃Cl | (CH₂)₅NH (1:1) | - | 70.3 | 0.45 |
| 30 | Rh[(dppb)COD]BF₄^{c} | (CH₂)₅NH (1:1) | - | > 99.9 | 1.8 |
| 31 | Rh[(dppb)COD]BF₄^{c} | (CH₂)₅NH (2:1) | - | > 99.9 | 1.9 |
| 32 | Rh[(dppb)COD]BF₄^{c} | (CH₂)₅NH (1:1) | TsOH (20:1) | > 99.9 | 4.8 |
| 33 | Rh[(DPOE)COD]BF₄^{d} | (CH₂)₅NH (1:1) | - | > 99.9 | 6.8 |
| 34 | Rh[(DPOE)COD]BF₄^{d} | (CH₂)₅NH (1:1) | TsOH (20:1) | > 99.9 | 3.2 |

| | | | | | |
|---|---|---|---|---|---|
| ^{a}Bedingungen siehe Tabelle 1; ^{b}Umsatz nach 20 Stdn.; ^{c}dppb = 1,4-Bis(diphenylphosphino)butan; ^{d}DPOE = 1,2-Bis(diphenylphosphinoxy)ethan. * Vergleichsbeispiel | | | | | |

**Tabelle 5. Enantioselektive Synthese von N-Benzylphenylalanin^{a} (Vergleichsbeispiele)**

| | | | | |
|---|---|---|---|---|
| 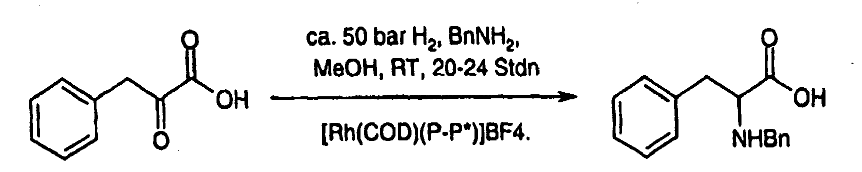 | | | | |

| **Beispiel** | **Ligand (P-P^{a})** | **Ausbeute** | **Ee^{b}** | **Konfiguration** |
|---|---|---|---|---|
| 41 | ^{c} | 59 | 38 | *R* |
| 42 | R-DIOP^{d} | 51 | 10 | *S* |
| 43 | *R*-Bdpch^{e} | 58 | 17 | *R* |
| 44 | *R*-Cyclopentyl-ppp' | 63 | 12 | *S* |

| | | | | |
|---|---|---|---|---|
| ^{a}Bedingungen siehe Beispiel 40; ^{b}GC-Analyse auf der chirale Säule L-Chirasil-Val; ^{c}(2R,3R,5R,6R)-2,3-Dimethoxy-2,3-dimethyl-5,6-bis(diphenylphosphinomethyl)-1,4-dioxan; ^{d}(4*R*,5*R*)-4,5-Bis-(diphenylphosphinomethyl)-2,2-dimethyl-1,3-dioxolan; ^{e}(1*R*,2*R*)-1,2-Bis (diphenylphosphinoxy)cyclohexan; ¹(*2R)*-*1*-[[(diphenylphosphino)(cyclopenthyl)amino]methyl]-2-diphenylphosphinoxy-3-(1-naphthalenyloxy)propan | | | | |

## Patentansprüche

1. Verfahren zur Herstellung von Aminen der Formel (III) 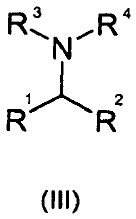 durch Umsetzung einer Verbindung der Formel (I) 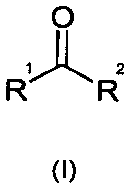 mit Ammoniak oder einem sekundären Amin der Formel (II) 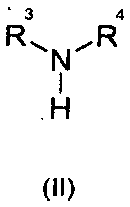 worin die Reste
R¹ bis R⁴ unabhängig voneinander aus der Gruppe von Wasserstoff, (C₁-C₂₄)-Alkyl, (C₂-C₂₄)-Alkenyl, (C₂-C₂₄)-Alkinyl, (C₆-C₁₀)-Aryl, CF₃, CN, COOH, COOM, wobei M ein Kation ausgewählt aus der Gruppe Li⁺, Na⁺, K⁺, Mg2⁺, Ca²⁺, NH₄⁺, N(C₁-C₁₀-Alkyl)₄⁺, N(C₁-C₁₀-Alkyl/C₆-C₁₀-Aryl)₄⁺ darstellt, CHO, SO₃H, COO-Alkyl-(C₁-C₈), CONH₂, CONHAlkyl-(C₁-C₈), CONAlkyl₂-(C₁-C₈), CO-Alkyl-(C₁-C₈), CO-Phenyl, COO-Phenyl, COO-Aryl-(C₆-C₁₀), CO-Aryl-(C₆-C₁₀), P(Aryl)₂, PAlkyl₂-(C₁-C₈), PO(Aryl)₂, POAlkyl₂-(C₁-C₄), PO₃H₂, POAlkyl-(C₁-C₄)(O-Alkyl-(C₁-C₆)), PO(O-Alkyl-(C₁-C₆))₂, SO₃-Alkyl-(C₁-C₄), SO₂-Alkyl-(C₁-C₆), SO-Alkyl-(C₁-C₆) oder Si(Alkyl)₃-(C₁-C₈). und/oder R³ und R⁴ unabhängig voneinander aus der Gruppe O-Alkyl-(C₁-C₈), OCO-Alkyl-(C₁-C₈), O-Aryl, Fluor, OH, NH₂, NH-Alkyl-(C₁-C₈), N-Alkyl₂-(C₁-C₈), NHCO-Alkyl-(C₁-C₄), NHCOO-Alkyl-(C₁-C₄), NHAryl-(C₆-C₁₀), ausgewählt werden, wobei R³, R⁴ gleichzeitig Wasserstoff ist oder R³, R⁴ nicht Wasserstoff ist,
wobei Alkyl im Sinne der vorliegenden Erfindung für einen nichtverzweigten oder verzweigten Rest steht, Alkenyl für einen olefinischen Kohlenwasserstoff, Alkinyl für einen Acetylenkohlenwasserstoff und Aryl für einen aromatischen Rest, welcher auch ein Aromat mit ein bis vier Heteroatomen sein kann ausgewählt aus der Gruppe der Stickstoff-, Schwefel- oder Sauerstoffatom steht,
sowohl Alkyl, Alkenyl, Alkinyl als auch Aryl können Substituenten die unabhängig voneinander Wasserstoff, O-Alkyl-(C₁-C₈), OCO-Alkyl-(C₁-C₈), O-Phenyl, Phenyl, Aryl (C₆-C₁₀), Fluor, Chlor, Brom, lod, OH, CF₃, CN, COOH, COOM, wobei M ein Kation ausgewählt aus der Gruppe Li⁺, Na⁺, K⁺, Mg²⁺, Ca²⁺, NH₄⁺, N(C₁-C₁₀-Alkyl)₄⁺, N(C₁-C₁₀-Alkyl/C₆-C₁₀-Aryl)₄⁺ darstellt, CHO, SO₃H, NH₂, NH-Alkyl-(C₁-C₈), N-Alkyl₂-(C₁-C₈), NHCO-Alkyl-(C₁-C₄), COO-Alkyl-(C₁-C₈), CONH₂, CO-Alkyl-(C₁-C₈), NHCOH, NHCOO-Alkyl-(C₁-C₄), CO-Phenyl, COO-Phenyl, COO-Aryl-(C₆-C₁₀), CO-Aryl-(C₆-C₁₀), CHCH-CO₂-Alkyl-(C₁-C₈), P(Aryl)₂, CHCHCO₂H, P-Alkyl₂-(C₁-C₈), PO-Aryl₂, POAlkyl₂-(C₁-C₄), PO₃H₂, POAlkyl-(C₁-C₄)(O-Alkyl-(C₁-C₆)), PO(O-Alkyl-(C₁-C₆))₂, SO₃-Alkyl-(C₁-C₄), SO₂-Alkyl-(C₁-C₆), SO-Alkyl-(C₁-C₆) oder Si(Alkyl)₃-(C₁-C₈) bedeuten, tragen können und wobei
R³ und R⁴ durch kovalente Bindungen verknüpft sein können, so dass R³ und R⁴ jeweils einen vier- bis achtgliedrigen Ring bilden,
in Gegenwart von Wasserstoff und einem homogenen Katalysatorsystem enthaltend mindestens ein Metallatom ausgewählt aus der Gruppe Rh und einen oder mehrere bidentate achirale oder chirale Ligand, wobei als Ligand 1,4-Bis(diphenylphosphino)butan), 1,4-Bis(dicyclohexylphosphino)butan, 2-Diphenylphosphinomethyl-4-diphenylphosphino-1-tert-butoxycarbonylpyrrolidin, 2,3-O-isopropyliden-2,3-dihydroxy-1,4-bis(diphenylphosphino)butane, (2R,3R,5R,6R)-2,3-Dimethoxy-2,3-dimethyl-5,6-bis(diphenylphosphinomethyl)-1,4-dioxan, 2,2'-Bis[[bis(3-sulfophenyl)phosphino]methyl]- 4,4',7,7'-tetrasulfo-1,1'-binapthyl Octanatriumsalz, Diphosphinit-Liganden auf Kohlenhydratbasis, 1,2-Bis(diphenylphosphinoxy)ethan), (1*R*,2*R*)-(trans)-1,2-Bis-(diphenylphosphinoxy)cyclohexan, (*2R*)-*1*-[[(diphenylphosphino)(cyclopenthyl)-amino]methyl]-2-diphenylphosphinoxy-3-(1-naphthalenyloxy)propan und/oder (4*S*)-2-(2-(Diphenylphosphino)phenyl)-4-isopropyl-1,3-oxazoline verwendet werden und wobei als Lösungsmittel Alkohole, Wasser und Gemische derselben eingesetzt werden.

2. Verfahren nach einem der vorherigen Ansprüche **dadurch gekennzeichnet, dass** Edukte der Formeln (I) und/oder (II) eingesetzt werden, deren Substituenten R¹ bis R⁴ unabhängig voneinander Wasserstoff, (C₁-C₁₂)-Alkyl, (C₂-C₁₂)-Alkenyl, (C₂-C₁₂)-Alkinyl, (C₆-C₁₀)-Aryl, CF₃, CN, COOH, COOM, wobei M ein Kation aus der Gruppe Li⁺, Na⁺, K⁺, Mg²⁺, Ca²⁺, NH₄⁺, N(C₁-C₁₀-Alkyl)₄⁺, N(C₁-C₁₀-Alkyl/C₆-C₁₀-Aryl)₄⁺)darstellt, COO-Alkyl-(C₁-C₈), CONH₂, CONHAlkyl-(C₁-C₈), CONAlkyl₂-(C₁-C₈), CO-Alkyl-(C₁-C₈), CO-Phenyl, COO-Phenyl, COO-Aryl-(C₆-C₁₀), CO-Aryl-(C₆-C₁₀), PO(Aryl-C₆-C₁₀)₂, POAlkyl₂-(C₁-C₄), PO₃H₂, PO(Alkyl-(C₁-C₄))(OAlkyl-(C₁-C₄)), PO(O-Alkyl-(C₁-C₆))₂ oder Si(Alkyl)₃-(C₁-C₈) und/oder R³ und R⁴ unabhängig voneinander aus der Gruppe O-Alkyl-(C₁-C₈), OCO-Alkyl-(C₁-C₈), O-Aryl (C₆-C₁₀), OH, NH₂, NH-Alkyl-(C₁-C₈), N-Alkyl₂-(C₁-C₈), NHCO-Alkyl-(C₁-C₄), NHCOO-Alkyl-(C₁-C₄), NHAryl-(C₆-C₁₀), ausgewählt werden,
wobei Alkyl einen nichtverzweigten oder verzweigten Rest, Alkenyl einen olefinischen Kohlenwasserstoff, Alkinyl einen Acetylenkohlenwassersoff und Aryl einen aromatischen Rest, welcher auch ein Aromat mit 1-4 Heteroatomen aus der Gruppe N, O und/oder S sein kann, bedeutet,
und wobei sowohl Alkyl, Alkenyl und Alkinyl als auch Aryl Substituenten tragen können, die unabhängig voneinander Wasserstoff, O-Alkyl-(C₁-C₈), OCO-Alkyl-(C₁-C₈), O-Phenyl, Phenyl, Aryl- C₆-C₁₀, Fluor, Chlor, Brom, lod, OH, SiAlkyl₃-(C₁-C₈), CF₃, CN, COOH, COOM, wobei M ein einwertiges Kation ausgewählt ist aus der Gruppe Na, K, Rb, Cs, NH₄, N(C₁-C₁₀-Alkyl)₄, N(C₁-C₁₀-Alkyl/ C₆-C₁₀-Aryl)₄ darstellt, SO₃H, N-Alkyl₂-(C₁-C₈), SO₂-Alkyl-(C₁-C₆), SO-Alkyl-(C₁-C₆), NHCO-Alkyl-(C₁-C₄), COO-Alkyl-(C₁-C₈), CONH₂, CO-Alkyl-(C₁-C₈), CO-Phenyl, COO-Phenyl, COO-Aryl-(C₆-C₁₀), CO-Aryl-(C₆-C₁₀), PO-Phenyl₂, POAlkyl₂-(C₁-C₄), PO₃H₂, POAlkyl-(C₁-C₄)(O-Alkyl-(C₁-C₆)), PO(O-Alkyl-(C₁-C₆))₂, Si(Alkyl)₃-(C₁-C₈) bedeuten, wobei Alkyl und Aryl oben genannte Bedeutung haben.

3. Verfahren nach einem der vorherigen Ansprüche, **dadurch gekennzeichnet, dass** Edukte der Formeln (I) und/oder (II) eingesetzt werden, bei denen R³ und R⁴ durch kovalente Bindungen verknüpft sind, so dass ein drei- bis neungliedriger Ring vorliegt.

4. Verfahren nach einem der vorherigen Ansprüche, **dadurch gekennzeichnet, dass** Alkyl für einen nichtverzweigten oder verzweigten aliphatischen oder cyclischen Kohlenwasserstoff und Aryl für einen aromatischen Rest steht.

5. Verfahren gemäß Anspruch 4, **dadurch gekennzeichnet, dass** sowohl Alkyl als auch Aryl Substituenten die unabhängig voneinander Wasserstoff, O-Alkyl-(C₁-C₈), O-Phenyl, Phenyl, Aryl, Fluor, Chlor, OH, SiAlkyl₃-(C₁-C₄), CF₃, CN, SO₃H, N-Alkyl₂-(C₁-C₄), CO-Phenyl, COO-Phenyl, COO-Aryl-(C₆-C₁₀), CO-Aryl-(C₆-C₁₀), PO-Phenyl₂, POAlkyl₂-(C₁-C₄), PO(O-Alkyl-(C₁-C₆))₂, Si(Alkyl)₃-(C₁-C₈) bedeuten, wobei Alkyl und Aryl oben genannte Bedeutung haben, aufweist.

6. Verfahren gemäß einem der vorherigen Ansprüche, **dadurch gekennzeichnet, dass** das erfindungsgemäße Verfahren bei einer Temperatur von -40 -100 °C durchgeführt wird.

7. Verfahren gemäß einem der vorherigen Ansprüche, **dadurch gekennzeichnet, dass** bei dem Verfahren weitere Additive eingesetzt werden.

8. Verfahren gemäß Anspruch 7, **dadurch gekennzeichnet, dass** das Verfahren mit Phosphin-Rhodium-Komplexen in Anwesenheit von Säuren durchgeführt wird

9. Verfahren gemäß einem der Ansprüche 1 bis 6, **dadurch gekennzeichnet, dass** das Verfahren mit Phosphinit-Rhodium-Katalysatoren ohne den Zusatz von Additiven abläuft.

10. Verfahren nach einem der vorherigen Ansprüche **dadurch gekennzeichnet, dass** der Wasserstoffanfangsdruck zwischen 0.1 und 300 bar liegt.

11. Verfahren gemäß einem der vorherigen Ansprüche, **dadurch gekennzeichnet, dass** das Katalysatorsystem in Mengen von 0.001 bis 5 mol-%, bezogen auf die Carbonylkomponente der Formel (I), eingesetzt wird.

## Claims

1. A process for preparing amines of the formula (III) 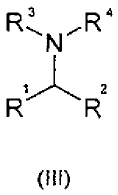 by reacting a compound of the formula (I) 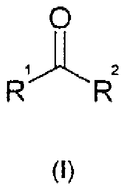 with ammonia or a secondary amine of the formula (II) 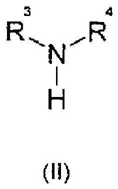 where the radicals
R¹ to R⁴ are selected independently from the group consisting of hydrogen, (C₁-C₂₄) -alkyl, (C₂-C₂₄)-alkenyl, (C₂-C₂₄) -alkynyl, (C₆-C₁₀) -aryl, CF₃, CN, COOH, COOM, where M is a cation selected from the group consisting of Li⁺, Na⁺, K⁺, Mg²⁺, Ca²⁺, NH₄⁺, N(C₁-C₁₀-alkyl)₄⁺, N(C₁-C₁₀-alkyl/C₆-C₁₀-aryl)₄⁺, CHO, SO₃H, COO-alkyl- (C₁-C₈), CONH₂, CONHalkyl-(C₁-C₈), CONalkyl₂- (C₁-C₈), CO-alkyl-(C₁-C₈), CO-phenyl, COO-phenyl, COO-aryl-(C₆-C₁₀), CO-aryl- (C₆-C₁₀), P(aryl)₂, Palkyl₂-(C₁-C₈), PO(aryl)₂, POalkyl₂- (C₁-C₄), PO₃H₂, POalkyl-(C₁-C₄) (O-alkyl-(C₁-C₆)), PO (O-alkyl-(C₁ - C₆))₂, SO₃-alkyl-(C₁-C₄), SO₂-alkyl-(C₁-C₆), SO-alkyl-(C₁-C₆) or Si(alkyl)₃-(C₁-C₈), and/or R³ and R⁴ are selected independently from the group consisting of O-alkyl-(C₁-C₈), OCO-alkyl-(C₁-C₈), O-aryl, fluorine, OH, NH₂, NH-alkyl-(C₁-C₈), N-alkyl₂-(C₁-C₈), NHCO-alkyl-(C₁-C₄), NHCOO-alkyl-(C₁-C₄), NHaryl-(C₆-C₁₀) where R³, R⁴ is simultaneously hydrogen or R³, R⁴ is not hydrogen,
where alkyl is, for the purposes of the present invention, an unbranched or branched radical, alkenyl is an olefinic hydrocarbon, alkynyl is an acetylenic hydrocarbon and aryl is an aromatic radical which may also be an aromatic containing from one to four heteroatoms selected from the group consisting of nitrogen, sulfur or oxygen atoms,
alkyl, alkenyl, alkynyl and also aryl may bear substituents which are independently hydrogen, O-alkyl-(C₁-C₈), OCO-alkyl-(C₁-C₈), O-phenyl, phenyl, aryl(C₆-C₁₀), fluorine, chlorine, bromine, iodine, OH, CF₃, CN, COOH, COOM, where M is a cation selected from the group consisting of Li⁺, Na⁺, K⁺, Mg²⁺, Ca²⁺, NH⁴⁺, N(C₁-C₁₀-alkyl)₄⁺, N(C₁-C₁₀-alkyl/C₆-C₁₀-aryl)4⁺, CHO, SO₃H, NH₂, NH-alkyl- (C₁-C₈), N-alkyl₂-(C₁-C₈), NHCO-alkyl- (C₁-C₄), COO-alkyl- (C₁-C₈),CONH₂, CO-alkyl-(C₁-C₈), NHCOH, NHCOO-alkyl-(C₁-C₄), CO-phenyl, COO-phenyl, COO-aryl-(C₆-C₁₀) , CO-aryl-(C₆-C₁₀), CHCH-CO₂-alkyl-(C₁-C₆), P(aryl)₂, CHCHCO₂H, P-alkyl₂-(C₁-C₈), PO-aryl₂, POalkyl₂-(C₁-C₄), PO₃H₂, POalkyl-(C₁-C₄)(O-alkyl-(C₁-C₆)), PO(O-alkyl-(C₁-C₆))₂, SO₃-alkyl-(C₁-C₄), SO₂-alkyl-(C₁-C₆), SO-alkyl-(C₁-C₆) or Si(alkyl)₃-(C₁-C₈),
R³ and R⁴ can be linked by covalent bonds so that R³ and R⁴ in each case form a four- to eight-membered ring,
in the presence of hydrogen and a homogeneous catalyst system comprising at least one metal atom selected from the group consisting of Rh and one or more bidentate achiral or chiral ligands,
where 1,4-bis(diphenylphosphino)butane, 1,4-bis(dicyclohexylphosphino)butane, 2-diphenylphosphinomethyl-4-diphenylphosphino-1-tert-butoxycarbonylpyrrolidine, 2,3-O-isopropylidene-2,3-dihydroxy-1,4-bis(diphenylphosphino)butane, (2R,3R,5R,6R)-2,3-dimethoxy-2,3-dimethyl-5,6-0bis(diphenylphosphinomethyl)-1,4-dioxane, 2,2'-bis[[bis(3-sulfophenyl)phosphino]methyl]-4,4',7,7'-tetrasulfo-1,1'-binaphthyl octasodium salt, diphosphinite ligands based on carbohydrates, 1,2-bis(diphenylphosphinoxy)ethane, (1R,2R)-(trans)-1,2-bis-(diphenylphosphinoxy)cyclohexane, (2R)-1-[[(diphenylphosphino)(cyclopenthyl)amino]methyl]-2-diphenylphosphinoxy-3-(1-naphthalenyloxy)propane and/or (4S)-2-(2-(diphenylphosphino)phenyl)-4-isopropyl-1,3-oxazoline are used as ligands where solvents used are alcohols, water and mixtures thereof.

2. The process as claimed in the preceding claim, wherein the starting materials of the formulae (I) and/or (II) used are ones whose substituents R¹ to R⁴ are selected, independently of one another, from hydrogen, (C₁-C₁₂)-alkyl, (C₂-C₁₂) -alkenyl, (C₂-C₁₂)-alkynyl, (C₆-C₁₀)-aryl, CF₃, CN, COOH, COOM, where M is a cation selected from the group consisting of Li⁺*,* Na⁺, K⁺, Mg²⁺, Ca²⁺, NH₄⁺, N(C₁-C₁₀-alkyl)₄⁺, N(C₁-C₁₀-alkyl/C₆-C₁₀-aryl)₄⁺, COO-alkyl-(C₁-C₈), CONH₂, CONHalkyl-(C₁-C₈),CONalkyl₂-(C₁-C₈), CO-alkyl-(C₁-C₈), CO-phenyl, COO-phenyl, COO-aryl-(C₆-C₁₀), CO-aryl-(C₆-C₁₀), PO (aryl-C₆-C₁₀)₂, POalkyl₂-(C₁-C₄), PO₃H₂, PO (alkyl-(C₁-C₄)) (Oalkyl- (C₁-C₄)), PO (O-alkyl- (C₁-C₆))₂ or Si(alkyl)₃-(C₁-C₈) and/or R³ and R⁴ are selected independently from the group consisting of O-alkyl-(C₁-C₈), OCO-alkyl-(C₁-C₈), O-aryl(C₆-C₁₀), OH, NH₂, NH-alkyl- (C₁-C₈), N-alkyl₂- (C₁-C₈), NHO-alkyl-(C₁-C₄), NHCOO-alkyl-(C₁-C₄), NHaryl-(C₆-C₁₀), where alkyl is an unbranched or branched radical, alkenyl is an olefinic hydrocarbon, alkynyl is an acetylenic hydrocarbon and aryl is an aromatic radical which may also be an aromatic containing 1-4 heteroatoms from the group consisting of N, O and/or S,
and alkyl, alkenyl and alkynyl and also aryl may bear substituents which are independently hydrogen, O-alkyl-(C₁-C₈), OCO-alkyl-(C₁-C₈), O-phenyl, phenyl, aryl-C₆-C₁₀, fluorine, chlorine, bromine, iodine, OH, Si-alkyl₃-(C₁-C₈), CF₃, CN, COOH, COOM, where M is a monovalent cation selected from the group consisting of Na, K, Rb, Cs, NH₄, N(C₁-C₁₀-alkyl)₄, N(C₁-C₁₀-alkyl/C₆-₁₀-aryl)₄, and SO₃H, N-alkyl₂-(C₁-C₈), SO₂-alkyl-(C₁-C₆), SO-alkyl-(C₁-C₆), NHCO-alkyl-(C₁-C₄), COO-alkyl-(C₁-C₈), CONH₂, CO-alkyl-(C₁-C₈), CO-phenyl, COO-phenyl, COO-aryl-(C₆-C₁₀), CO-aryl- (C₆-C₁₀), PO-phenyl₂, POalkyl₂- (C₁-C₄) , PO₃H₂, POalkyl- (C₁-C₄)(O-alkyl-(C₁-C₆)), PO(O-alkyl-(C₁-C₆))₂, Si(alkyl)₃-(C₁-C₈), where alkyl and aryl are as defined above.

3. The process as claimed in either of the preceding claims, wherein the starting materials of the formulae (I) and/or (II) used are ones in which R³ and R⁴ are linked by covalent bonds so as to form a three- to nine-membered ring.

4. The process as claimed in any of the preceding claims, wherein alkyl is an unbranched or branched aliphatic or cyclic hydrocarbon and aryl is an aromatic radical.

5. The process as claimed in claim 4, wherein both alkyl and aryl bear substituents which are independently hydrogen, O-alkyl-(C₁-C₈), O-phenyl, phenyl, aryl, fluorine, chlorine, OH, Si-alkyl₃-(C₁-C₄), CF₃, CN, SO₃H, N-alkyl₂-(C₁-C₄), CO-phenyl, COO-phenyl, COO-aryl-(C₆-C₁₀), CO-aryl-(C₆-C₁₀), PO-phenyl₂, PO-alkyl₂-(C₁-C₄), PO (O-alkyl (C₁-C₆))₂, Si((alkyl)₃-(C₁-C₈), where alkyl and aryl are as defined above.

6. The process as claimed in any of the preceding claims which is carried out at a temperature of -40-100°C.

7. The process as claimed in any of the preceding claims in which further additives are used.

8. The process as claimed in claim 7 carried out using phosphine-rhodium complexes in the presence of acids.

9. The process as claimed in any of claims 1 to 6 carried out using phosphinite-rhodium catalysts without the addition of additives.

10. The process as claimed in any of the preceding claims, wherein the initial hydrogen pressure is from 0.1 to 300 bar.

11. The process as claimed in any of the preceding claims, wherein the catalyst system is used in an amount of from 0.001 to 5 mol%, based on the carbonyl component of the formula (I).

## Revendications

1. Procédé de préparation d'amines de formule (III) 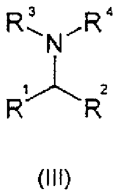 par transformation d'un composé de formule (I) 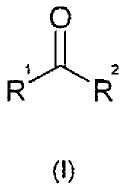 avec de l'ammoniaque ou une amine secondaire de formule (II) 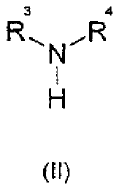 dans lesquelles les radicaux
R¹ à R⁴ sont choisis, indépendamment l'un de l'autre, dans le groupe constitué par l'hydrogène, alkyle en C₁ à C₂₄, alcényle en C₂ à C₂₄, alcynyle en C₂ à C₂₄, aryle en C₆ à C₁₀, CF₃, CN, COOH, COOM, où M représente un cation choisi dans le groupe constitué par Li⁺, Na⁺, K⁺, Mg²⁺, Ca²⁺, NH₄⁺, N- (alkyle en C₁ à C₁₀)₄⁺, N- (alkyle en C₁ à C₁₀/aryle en C₆ à C₁₀)₄⁺, CHO, SO₃H, COO-alkyle en C₁ à C₈, CONH₂, CONH-alkyle en C₁ à C₈, CON- (alkyle en C₁ à C₈)₂, CO-alkyle en C₁ à C₈, CO-phényle, COO-phényle, COO-aryle en C₆ à C₁₀, CO-aryle en C₆ à C₁₀, P(aryle)₂, P(alkyle en C₁ à C₈)₂, PO(aryle)₂, PO(alkyle en C₁ à C₄)₂, PO₃H₂, PO(alkyle en C₁ à C₄)-(O-alkyle en C₁ à C₆), PO(O-alkyle en C₁ à C₆)₂, SO₂-alkyle en C₁ à C₄, SO₂-alkyle en C₁ à C₆, SO-alkyle en C₁ à C₆ ou Si(alkyle en C₁ à C₈)₃ et/ou R³ et R⁴ sont choisis, indépendamment l'un de l'autre, dans le groupe constitué par O-alkyle en C₁ à C₈, OCO-alkyle en C₁ à C₈, O-aryle, fluor, OH, NH₂, NH-alkyle en C₁ à C₈, N-(alkyle en C₁ à C₈)₂, NHCO-alkyle en C₁ à C₄, NHCOO-alkyle en C₁ à C₄, NH-aryle en C₆ à C₁₀, où R³, R⁴ est simultanément hydrogène ou R³, R⁴ n'est pas hydrogène,
où alkyle représente, dans le sens de la présente invention, un radical non ramifié ou ramifié, alcényle représente un hydrocarbure oléfinique et alcynyle un hydrocarbure acétylénique et aryle représente un radical aromatique qui peut également être un aromatique comprenant un à quatre hétéroatomes choisis dans le groupe constitué par les atomes d'azote, de soufre ou d'oxygène,
alkyle, alcényle, alcynyle ainsi qu'aryle pouvant porter des substituants qui signifient, indépendamment l'un de l'autre, hydrogène, O-alkyle en C₁ à C₈, OCO-alkyle en C₁ à C₈, O-phényle, phényle, aryle en C₆ à C₁₀, fluor, chlore, brome, iode, OH, CF₃, CN, COOH, COOM, où M représente un cation choisi dans le groupe constitué par Li⁺, Na⁺, K⁺, Mg²⁺, Ca²⁺, NH₄⁺, N(alkyle en C₁ à C₁₀)₄⁺, N(alkyle en C₁ à C₁₀/aryle en C₆ à C₁₀)₄⁺, CHO, SO₃H, NH₂, NH-alkyle en C₁ à C₈, N- (alkyle en C₁ à C₈)₂, NHCO-alkyle en C₁ à C₄, COO-alkyle en C₁ à C₈, CONH₂, CO-alkyle en C₁ à C₈, NHCOH, NHCOO-alkyle en C₁ à C₄, CO-phényle, COO-phényle, COO-aryle en C₅ à C₁₀, CO-aryle en C₆ à C₁₀, CHCH-CO₂-alkyle en C₁ à C₈, P(aryle)₂, CHCHCO₂H, P- (alkyle en C₁ à C₈)₂, PO-aryle₂, PO(alkyle en C₁ à C₄) PO₃H₂, PO (alkyle en C₁ à C₄)-(O-alkyle en C₁ à C₆), PO(O-alkyle en C₁ à C₆)₂, SO₃-alkyle en C₁ à C₄, SO₂-alkyle en C₁ à C₆, SO-alkyle en C₁ à C₆ ou Si (alkyle en C₁ à C₈)₃ et où
R³ et R⁴ peuvent être liés par des liaisons covalentes, de telle manière que R³ et R⁴ forment à chaque fois un cycle à quatre jusqu'à huit chaînons,
en présence d'hydrogène et d'un système de catalyseur homogène, contenant au moins un atome de métal choisi dans le groupe constitué par Rh et un ou plusieurs ligands bidentates, non chiraux ou chiraux où on utilise comme ligand du 1,4-bis-(diphénylphosphino)butane, du 1,4-bis(dicyclo-hexylphosphino)butane, de la 2-diphénylphosphinométhyl-4-diphénylphosphino-1-tert-butoxycarbonylpyrrolidine, du 2,3-O-isopropylidène-2,3-dihydroxy-1,4-bis(diphényl-phosphino)butane, du (2R,3R,5R,6R)-2,3-diméthoxy-2,3-diméthyl-5,6-bis(diphénylphosphinométhyl)-1,4-dioxanne, le sel octasodique du 2,2'-bis[[bis(3-sulfophényl)-phosphino]méthyl]-4,4',7,7'-tétrasulfo-1,1'-binaphtyle, des ligands diphosphinite à base d'hydrates de carbone, du 1,2-bis(diphénylphosphinoxy)éthane, du (1R,2R)-(trans)-1,2-bis-(diphénylphosphinoxy)cyclohexane, du (2R)-1-[[(diphénylphosphino)(cyclopentyl)-amino]méthyl]-2-diphénylphosphinoxy-3-(7-naphtalényloxy)propane et/ou de la (4S)-2-(2-(diphénylphosphino)phényl)-4-isopropyl-1,3-oxazoline et où on utilise comme solvant des alcools, de l'eau et des mélanges de ceux-ci.

2. Procédé selon la revendication précédente, **caractérisé en ce qu'**on utilise des produits de départ des formules (I) et/ou (II), dont les substituants R¹ à R⁴ sont choisis, indépendamment l'un de l'autre, dans le groupe hydrogène, alkyle en C₁ à C₁₂, alcényle en C₂ à C₁₂, alcynyle en C₂ à C₁₂, aryle en C₆ à C₁₀, CF₃, CN, COOH, COOM, M représentant un cation choisi dans le groupe constitué par Li⁺, Na⁺, K⁺, Mg²⁺, Ca²⁺, NH₄⁺, N (alkyle en C₁ à C₁₀)₄⁺, N(alkyle en C₁ à C₁₀/aryle en C₆ à C₁₀)₄⁺, COO-alkyle en C₁ à C₈, CONH₂, CONH-alkyle en C₁ à C₈, CON-(alkyle en C₁ à C₈)₂, CO-alkyle en C₁ à C₈, CO-phényle, COO-phényle, COO-aryle en C₆ à C₁₀, CO-aryle en C₆ à C₁₀, PO (aryle en C₆ à C₁₀)₂, PO (alkyle en C₁ à C₄)₂, PO₃H₂, PO(alkyle en C₁ à C₄)-(O-alkyle en C₁ à C₄), PO(O-alkyle en C₁ à C₆)₂ ou Si(alkyle en C₁ à C₈)₃ et/ou R³ et R⁴ sont choisis, indépendamment l'un de l'autre, dans le groupe O-alkyle en C₁ à C₈, OCO-alkyle en C₁ à C₈, O-aryle en C₆ à C₁₀, OH, NH₂, NH-alkyle en C₁ à C₈, N-(alkyle en C₁ à C₈)₂, NHCO-alkyle en C₁ à C₄, NHCOO-alkyle en C₁ à C₄, NH-aryle en C₅ à C₁₀, où alkyle signifie un radical non ramifié ou ramifié, alcényle un hydrocarbure oléfinique, alcynyle un hydrocarbure acétylénique et aryle un radical aromatique, qui peut également être un aromatique comprenant 1 à 4 hétéroatomes du groupe N, O et/ou S,
et où alkyle, alcényle et alcynyle ainsi qu'aryle peuvent porter des substituants qui signifient, indépendamment l'un de l'autre, hydrogène, O-alkyle en C₁ à C₈, OCO-alkyle en C₁ à C₈, O-phényle, phényle, aryle en C₆ à C₁₀, fluor, chlore, brome, iode, OH, Si(alkyle en C₁ à C₈)₃, CF₃, CN, COOH, COOM, où M est un cation monovalent, choisi dans le groupe Na, K, Rb, Cs, NH₄, N (alkyle en C₁ à C₁₀)₄, N (alkyle en C₁ à C₁₀/aryle en C₆ à C₁₀)₄, SO₃H, N-(alkyle en C₁ à C₈)₂, SO₂-alkyle en C₁ à C₆, SO-alkyle en C₁ à C₆, NHCO-alkyle en C₁ à C₄, COO-alkyle en C₁ à C₈, CONH₂ , CO-alkyle en C₁ à C₈, CO-phényle, COO-phényle, COO-aryle en C₆ à C₁₀, CO-aryle en C₆ à C₁₀, PO-phênyle₂, PO-(alkyle en C₁ à C₄)₂, PO₃H₂, PO-(alkyle en C₁ à C₄)-(O-alkyle en C₁ à C₆), PO(O-alkyle en C₁ à C₆)₂, Si(alkyle en C₁ à C₈)₃, alkyl et aryle ayant la signification susmentionnée.

3. Procédé selon l'une quelconque des revendications précédentes, **caractérisé en ce qu'**on utilise des produits de départ de formules (I) et/ou (II), dans lesquelles R³ et R⁴ sont liés par des liaisons covalentes de telle manière qu'il s'agit d'un cycle à trois jusqu'à neuf chaînons.

4. Procédé selon l'une quelconque des revendications précédentes, **caractérisé en ce qu'**alkyle représente un hydrocarbure non ramifié ou ramifié, aliphatique ou cyclique et aryle représente un radical aromatique.

5. Procédé selon la revendication 4, **caractérisé en ce qu'**alkyle ainsi qu'aryle présentent des substituants qui signifient, indépendamment l'un de l'autre, hydrogène, O-alkyle en C₁ à C₈, O-phényle, phényle, aryle, fluor, chlore, OH, Si(alkyle en C₁ à C₄)₃, CF₃, CN, SO₃H, N-(alkyle en C₁ à C₄)₂, CO-phényle, COO-phényle, COO-aryle en C₆ à C₁₀, CO-aryle en C₆ à C₁₀, PO-phényle₂, PO(alkyle en C₁ à C₄)₂, PO(O-alkyle en C₁ à C₆)₂, Si(alkyle en C₁ à C₈)₃, alkyle et aryle ayant la signification susmentionnée.

6. Procédé selon l'une quelconque des revendications précédentes, **caractérisé en ce que** le procédé selon l'invention est réalisé à une température de -40 à -100°C.

7. Procédé selon l'une quelconque des revendications précédentes, **caractérisé en ce qu'**on utilise d'autres additifs dans le procédé.

8. Procédé selon la revendication 7, **caractérisé en ce que** le procédé est réalisé avec des complexes phosphine-rhodium en présence d'acides.

9. Procédé selon l'une quelconque des revendications 1 à 6, **caractérisé en ce que** le procédé se déroule avec des catalyseurs phosphinite-rhodium sans addition d'additifs.

10. Procédé selon l'une quelconque des revendications précédentes, **caractérisé en ce que** la pression d'hydrogène de départ est située entre 0,1 et 300 bars.

11. Procédé selon l'une quelconque des revendications précédentes, **caractérisé en ce que** le système de catalyseur est utilisé en des quantités de 0,001 à 5% en mole par rapport au composant carbonyle de formule (I).
